Europäisches Patentamt

(19) **European Patent Office**

**Office européen des brevets**

(11) Veröffentlichungsnummer: **0 187 903**

**B1**

(12) # EUROPÄISCHE PATENTSCHRIFT

(45) Veröffentlichungstag der Patentschrift:
04.01.89

(21) Anmeldenummer: **85113574.9**

(22) Anmeldetag: **25.10.85**

(51) Int. Cl.⁴: **A 61 F 2/36**

(54) **Hüftgelenkprothese.**

(30) Priorität: **13.12.84 CH 5928/84**

(43) Veröffentlichungstag der Anmeldung:
**23.07.86 Patentblatt 86/30**

(45) Bekanntmachung des Hinweises auf die Patenterteilung:
**04.01.89 Patentblatt 89/1**

(84) Benannte Vertragsstaaten:
**AT DE FR GB IT**

(56) Entgegenhaltungen:
**EP-A-0 066 879**
**EP-A-0 066 880**
**DE-C-362 413**
**US-A-1 806 506**
**US-A-2 947 308**
**US-A-3 102 536**

(73) Patentinhaber: **GEBRÜDER SULZER AKTIENGESELLSCHAFT**, Zürcherstrasse 9, CH-8401 Winterthur (CH)

(72) Erfinder: **Frey, Otto, Wallrütistrasse 56, CH- 8400 Winterthur (CH)**
Erfinder: **Koch, Rudolf, Oberdorfstrasse 229, CH-8267 Berlingen (CH)**

(74) Vertreter: **Dipl.- Ing. H. Marsch Dipl.- Ing. K. Sparing Dipl.- Phys.Dr. W.H. Röhl Patentanwälte, Rethelstrasse 123, D-4000 Düsseldorf 1 (DE)**

EP 0 187 903 B1

**Beschreibung**

Die Erfindung betrifft eine Hüftgelenksprothese mit, mindestens im distalen Bereich, geradem, im Querschnitt runden und durchmesserinvarianten Schaft, an den für die Aufnahme des Gelenkkopfes proximal unter einem Winkel ein Prothesenhals angesetzt ist, der von einem zur Abstützung auf dem kortikalen Rand des resezierten Knochens bestimmten Kragen umgeben ist.

In den letzten Jahren ist man bestrebt, die die Belastungskräfte auf den Femurknochen übertragende Verankerung von Hüftgelenksprothesen möglichst nahe dem Femur- bzw. dem Gelenkkopf vorzunehmen (WO 83/02 555); dabei wird ein Teil dieser diese Kräfte über einen auf den kortikalen Rand der Resektionsöffnung abgestützten Kragen weitergeleitet, wobei der Schaft im Bereich der Diaphyse sich weder medial noch lateral auf der Kortikalis abstützen soll, damit er in gewissem Umfang axial verschiebbar bleibt.

Es ist weiterhin an sich bekannt (EP-A-0 086 879 und 0 086 880), im Durchmesser invariante Schäfte mit Hilfe einer Verankerungshülse so im Knochen zu lagern, dass sie in radialer Richtung zentriert, in axialer Richtung jedoch beweglich sind.

Bei proximal fixierten Prothesen müssen der Resektionsschnitt am Knochen und damit der Abstand Gelenkkopf - Auflagefläche des Kragens genau passen, wenn nicht Fehlstellungen des Gelenkkopfes relativ zum übrigen Skelett, beispielsweise eine falsche Höhe des Kopfes relativ zum grossen Trochanter oder ein Aufliegen der Prothese auf der Kortikalis im Diaphysen-Bereich, auftreten sollen.

Aufgabe der Erfindung ist es, einerseits Zentrierung und axiale Verschiebbarkeit des distalen Schaftbereichs zu verbessern, und andererseits die Arbeit des Operateurs zu erleichtern, indem für die Lage des Resektionsschnittes ein gewisser Spielraum zugelassen wird. Die Lösung dieser zweifachen Aufgabe erfolgt dadurch, dass der Schaft im distalen Bereich mit Hilfe einer Verankerungshülse im Knochen axial beweglich und zentrierbar ist, und dass ferner der Abstand des den Prothesenhals umgebenden Kragens vom Mittelpunkt des Gelenkkopfes in einem gewissen Bereich einstellbar ist.

Die Verwendung einer Führungshülse für den distalen Schaftbereich trennt die Schaftoberfläche vom umgebenden Knochen bzw. Zementbett, so dass Knochenreizungen oder Beeinträchtigungen der Verbindung Schaft / Zementbett durch axiale Verschiebungen zumindest reduziert werden.

Durch die Einstellbarkeit der Auflagefläche des Kragens relativ zum Mittelpunkt des Gelenkkopfes und damit auch relativ zu jedem beliebigen anderen Bezugspunkt am Schaft oder Prothesenhals können Ungenauigkeiten in der Lage des Resektionsschnittes relativ zur Prothese ausgeglichen oder vermindert werden.

Eine bevorzugte Ausführungsform der Erfindung ergibt sich, wenn an den Kragen eine in Längsrichtung geschlitzte Hülse angesetzt ist, die innen mit einem Feinstgewinde oder einer Feinstverzahnung zum Eingriff in ein entsprechendes Gewinde bzw. eine entsprechende Verzahnung am Prothesenhals versehen ist; zusätzlich kann bei dieser Konstruktion ein Sicherungsring vorgesehen sein, der auf ein Aussengewinde der Hülse geschraubt wird, und dessen Bohrung mindestens im Endbereich konisch verläuft.

Im folgenden wird die Erfindung anhand eines Ausführungsbeispiels im Zusammenhang mit der Zeichnung näher erläutert.

Fig. 1    zeigt die neue Prothese als Implantat in einem in Längsrichtung geschnittenen Femurknochen;

Fig. 2    ist teilweise im Schnitt eine vergrösserte Darstellung des Kragenbereichs der Prothese nach Fig. 1, während

Fig. 3    schliesslich in starker Vergrösserung das Detail A aus Fig. 2 wiedergibt.

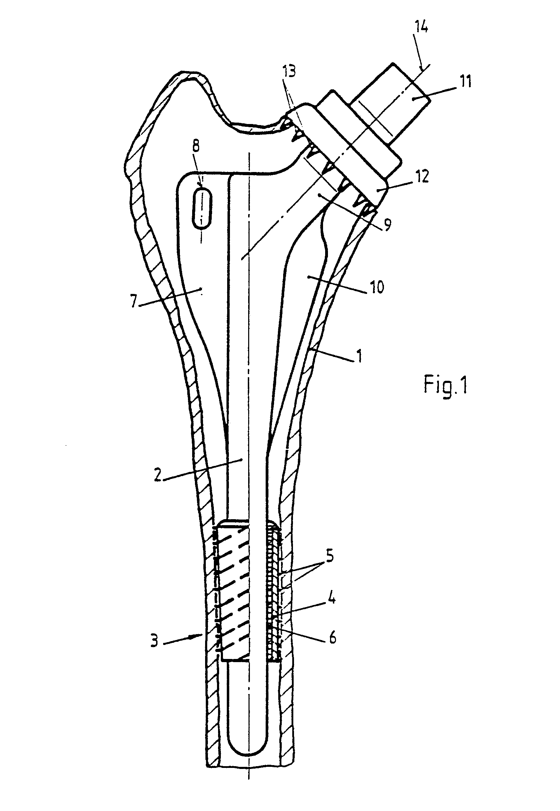

Im Femurknochen 1 (Fig. 1) ist zur Aufnahme des in seinem distalen Bereich mit einem konstanten Durchmesser versehenen, geraden Prothesenschaftes 2 eine Führungshülse 3 verankert. Diese weist einen metallenen Aussenmantel 4, beispielsweise aus Titan, auf; auf seiner Mantelfläche ist der Aussenmantel 4 mit einem scharfkantigen selbstschneidenden Gewinde 5 versehen, mit dem er in den kortikalen Knochen eingeschraubt wird. Der Aussenmantel 4 umschliesst eine Innenhülse 6 aus Polyäthylen, die in den Aussenmantel 4 eingepresst ist. Sie dient dazu, ein direktes Reiben der beiden Metallflächen von Aussenmantel 4 und distalem Bereich des im Quershnitt beispielsweise kreiszylindrischen Schaftes 2 zu vermeiden. Ihr Hohlraum ist dabei an den Durchmesser des distalen Schaftbereichs so angepasst, dass dieser in der Innenhülse 6 von Hand gleitend bewegt werden kann, ohne dass er in der Hülse 6 "taumelt".

Im proximalen Berich ist an den sich leicht erweiternden Schaft 2 lateral ein Trochanterflügel 7 angesetzt, der eine Ausziehöse 8 hat. Medial ist in dem Bogen, mit dem der Schaft 2 in einen Prothesenhals 9 übergeht, eine weitere flügelartige Rippe 10 vorgesehen. Beide Flügelelemente 7 und 10 dienen als Verdrehsicherung gegen unbeabsichtigte Rotationen der implantierten Prothese.

Der Prothesenhals 9, der in einem konischen Zapfen 11 zur Aufnahme des nicht gezeigten Gelenkkopfes endet, trägt einen relativ weit ausladenden Kragen 12; über diesen Kragen 12, der mit dornenartigen Ansätzen 13 in das kortikale Gewebe des Femurs 1 eindringt, werden Belastungskräfte von der Prothese auf den Knochen übertragen. Da - wie bereits erwähnt - zwischen den Flügeln 7 und 10 und dem harten kortikalen Knochengewebe ein Spiel vorhanden sein soll, ist der Kragen 12 erfindungsgemäss in Richtung der Prothesenhalsachse 14 einstellbar.

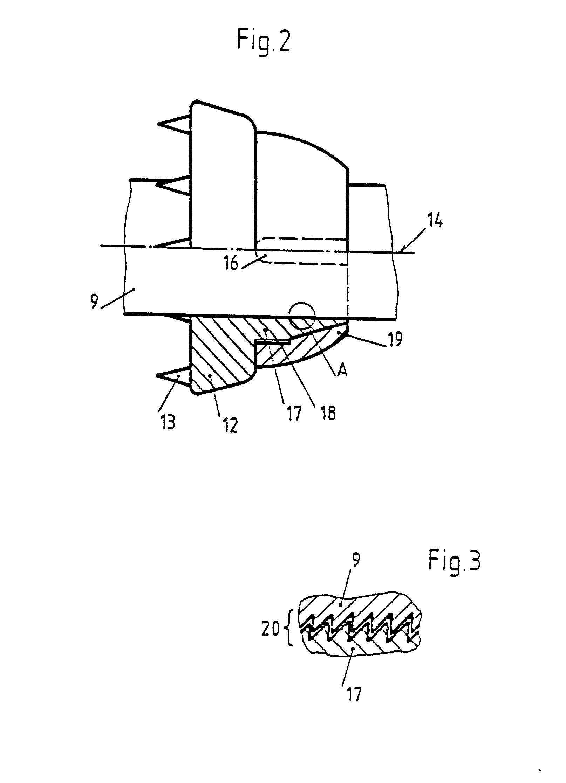

Dafür ist an den Kragen 12 zum Gelenkkopf hin eine mit mindestens einem Schlitz 16 (Fig. 2) versehene Hülse 17 angesetzt, die im kragennahen Bereich ein Aussengewinde 18 trägt. Auf dieses wird ein Sicherungsring 19 aufgeschraubt, durch den die Hülse 17 auf dem Prothesenhals 9 festgeklemmt wird. Um diesen Klemmsitz zu gewährleisten, verlaufen Hülse 17 und Ring 19 zum Gelenkkopf hin konisch.

Eine genaue Einstellung und ein verbesserter Sitz des Kragens 12 auf dem Prothesenhals 9 wird erreicht durch eine in Fig. 3 in stark vergrössertem Masse gezeigte Feinstverzahnung 20 auf jeder der beiden Oberflächen, die auch als Gewinde ausgebildet sein kann. Unter "Feinstverzahnung" werden dabei Zahnhöhen bis zu 0,5 mm verstanden.

## Patentansprüche

1. Hüftgelenkprothese mit, mindestens im distalen Bereich, geradem, im Querschnitt runden und durchmesserinvarianten Schaft (2), an den für die Aufnahme des Gelenkkopfes proximal unter einem Winkel ein Prothesehals (9) angesetzt ist, der von einem zur Abstützung auf dem kortikalen Rand des resezierten Knochens bestimmten Kragen (12) umgeben ist, dadurch gekennzeichnet, dass der Schaft im distalen Bereich mit Hilfe einer Verankerungshülse im Knochen axial beweglich und zentrierbar ist, und dass ferner der Abstand des den Prothesenhals (9) umgebenden Kragens (12) vom Mittelpunkt des Gelenkkopfes in einem gewissen Bereich einstellbar ist.

2. Hüftgelenksprothese nach Anspruch 1, dadurch gekennzeichnet, dass an den Kragen (12) eine in Längsrichtung geschlitzte Hülse (17) angesetzt ist, die innen mit einem Feinstgewinde oder einer Feinstverzahnung (20) zum Eingriff in ein entsprechendes Gewinde bzw. eine entsprechende Verzahnung (20) am Prothesenhals (9) versehen ist.

3. Hüftgelenksprothese nach Anspruch 1 oder 2, dadurch gekennzeichnet, dass die Hülse (17) aussen ein Gewinde (18) trägt zur Aufnahme eines Sicherungsringes (19), dessen Bohrung mindestens im Endbereich konisch verläuft.

## Claims

1. A hip joint replacement having at least in the distal zone a straight, circular-section and fixed-diameter stem (2) which merges proximally at an angle into a neck prosthesis (9) adapted to receive the head of the joint, a collar (12) which is adapted to bear on the cortical edge of the resected bone extending around the neck prosthesis (9) characterised in that in the distal region the stem is so disposed as through the agency of an anchoring sleeve to be axially mobile and centrable in the bone, and the distance between the collar (12) and the centre of the joint head is to some extent adjustable.

2. A hip joint replacement according to claim 1, characterised in that a longitudinally slotted sleeve (17) is attached to the collar (12) and has internally a very fine screw thread or very fine toothing (20) engageable in companion screw threading or toothing (20) on the neck prosthesis (9).

3. A hip joint replacement according to claim 1 or 2, characterised in that the sleeve (17) has an external screw thread (18) adapted to receive a securing ring (19) whose bore extends conically at least in the end zone.

## Revendications

1. Prothèse coxofémorale présentant une tige (2) qui est rectiligne au moins dans la région distale, est de section ronde et de diamètre constant, et à laquelle est attenant, selon un certain angle dans la région proximale, un col (9) de prothèse conçu pour recevoir la tête d'articulation, et entouré par une collerette (12) destinée à assurer l'appui sur le bord cortical de l'os dans lequel une résection a été pratiquée, caractérisée par le fait que la tige est centrable et mobile axialement dans l'os, dans la région distale, à l'aide d'une douille d'ancrage; et par le fait que, par ailleurs, la distance comprise entre la collerette (12) entourant le col (9) de la prothèse, et le centre de la tête d'articulation, est réglable dans une certaine plage.

2. Prothèse coxofémorale selon la revendication 1, caractérisée par le fait qu'une douille (17) fendue dans le sens longitudinal, ménagée dans la continuité directe de la collerette (12), est intérieurement pourvue d'un filetage fin ou d'une denture fine (20) en vue de son engagement dans un filetage correspondant, ou respectivement dans une denture correspondante (20) sur le col (9) de la prothèse.

3. Prothèse coxofémorale selon la revendication 1 ou 2, caractérisée par le fait que la douille (17) porte extérieurement un filetage (18), destiné à recevoir une bague d'arrêt (19) dont le perçage s'étend coniquement, au moins dans la zone extrême.

Fig.1

Fig.2

14

16

9

19

A

13    12    17    18

Fig.3

9

20 {

17